# EUROPEAN PATENT SPECIFICATION

(11) **EP 1 517 982 B1**
(45) Date of publication and mention of the grant of the patent: **16.02.2011**
(21) Application number: 03762192.7
(22) Date of filing: 27.06.2003
(51) Int. Cl.: C11D 7/32, C11D 17/04

(54) **IONIC LIQUID BASED PRODUCTS AND METHOD OF USING THE SAME**
PRODUKTE AUF BASIS VON IONISCHEN FLÜSSIGKEITEN UND VERFAHREN ZU DEREN VERWENDUNG
PRODUITS A BASE DE LIQUIDE IONIQUE ET LEUR PROCEDE D'UTILISATION

(30) Priority: 28.06.2002 US 392735 P
(43) Date of publication of application: 30.03.2005
(73) Proprietor: THE PROCTER & GAMBLE COMPANY, Cincinnati, OH 45202 (US)
(72) Inventor: PRICE, Kenneth, Nathan, Wyoming, OH 45215 (US); HARTSHORN, Richard, Timothy, Lawrenceburg Indiana 47025 (US); ROHRBAUGH, Robert, Henry, Hamilton, OH 45011 (US); SCHEPER, William Michael, Guilford, IN 47022-8772 (US); SHOWELL, Michael, Stanford, Cincinnati, OH 45231 (US); BAKER, Keith, Homer, Cincinnati, OH 45241 (US); SIVIK, Mark, Robert, Mason, OH 45040 (US); SCHEIBEL, Jeffrey, John, Loveland, OH 45140 (US); GARDNER, Robb, Richard, Cincinnati, OH 45002 (US); REDDY, Pramod, Kakumanu, West Chester, OH 45069 (US); AIKEN, John, Davis, III, Cincinnati, OH 45208 (US); ADDISON, Michael, Crombie, 47 Clousden Grange, Newcastle, Tyne and Wear NE12 0YX (GB)
(74) Representative: Vercruysse, Nicolas
(86) International application number: PCT/US2003/020448
(87) International publication number: WO 2004/003120

(56) References cited:
- WO-A-01/77486
- WO-A-02/26701
- US-A- 6 048 388
- WILKES J S ET AL: "AIR AND WATER STABLE 1-ETHYL-3-METHYLIMIDAZOLIUM BASED IONIC LIQUIDS" JOURNAL OF THE CHEMICAL SOCIETY, CHEMICAL COMMUNICATIONS, CHEMICAL SOCIETY. LETCHWORTH, GB, no. 13, 1992, pages 965-967, XP002044780 ISSN: 0022-4936
- HOLBREY J D ET AL: "IONIC LIQUIDS" CLEAN PRODUCTS AND PROCESSES, SPRINGER, BERLIN, DE, vol. 1, 1999, pages 223-236, XP001000207 ISSN: 1435-2974

## Description

### FIELD OF THE INVENTION

The present invention relates to ionic liquids, ionic liquid based products and methods of using such ionic liquids, products and/or compositions.

### BACKGROUND OF THE INVENTION

In recent years, ionic liquids have been extensively evaluated as environmental-friendly or "green" alternatives to conventional organic solvents for a broad range of organic synthetic applications. In addition, ionic liquids have also been used in organic synthesis applications as catalysts.

Ionic liquids also have applications in electrochemistry, for example, in fuel cells, electrodeposition processes and other electrochemical applications.

Additionally, ionic liquids have been shown to be effective in applications where water-based chemistry can be problematic (for example, applications involving proton transfer or nucleophilicity), or in applications where certain coordination chemistry could have a damaging effect on the substrates involved.

A broad range of ionic liquids have been investigated in the past. One widely studied class of ionic liquids includes imidazolinium salts, such as BMIM/PF6 (butylmethylimidazolinium hexafluorophosphate)

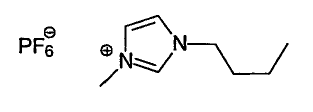

Other well known ionic liquid include N-1-ethyl 3-methylimidazolinum chloride aluminum (III) chloride, which is usually referred to as [emim]Cl-AlCl3; and N-butyl pyridinium chloride aluminum (III) chloride, which is usually referred to as [Nbupy] Cl-AlCl3.

Conventional applications of these and similar ionic liquids for a wide range of chemical processes are described in *"*Ionic Liquid" by J.D. Holbrey and K.R. Seddon, and in Clean Products and Processes, Vol. 1, pp. 223-236 (1999).

In addition to chemical processes, ionic liquids have also been used as microbiocides/plant growth regulators, as described in FR 2434156; as antistatic agents, as described in JP10-265674 and U.S. Patent No. 3,282,728; and as fruit and vegetable produce treating agents, as described in WO 01/19200.

Other uses of ionic liquids are disclosed in U.S. Patent 6,048,388 as a component of an ink composition; and in J. Am. Chem. Soc. Vol 124, pp. 4974-4975 (2002) as an agent to dissolve cellulose. WO-A-0 177 486 discloses ionic liquids for removing scale from a wellbore.

Nothing in the prior art suggests the use of ionic liquids in surface or air treating compositions for consumer products and/or industrial products. Further, nothing in the prior art teaches ionic liquid mixtures/cocktails that comprise a mixture of different ionic liquid components.

Therefore, it is desirable to provide compositions containing ionic liquids suitable for surface treating or air treating compositions. It is also desirable that such compositions be suitable for consumer applications (e.g., for the house or for the automobile) and/or industrial applications.

It is further desirable that such compositions contain a mixture of ionic liquids to enhance the desired benefits provided by several ionic liquids.

### SUMMARY OF THE INVENTION

The present invention provides ionic liquids, ionic liquid based products/compositions and methods for using them.

In one aspect of the present invention, a surface or air treating composition comprising an ionic liquid as defined in claim 1 is provided.

In another aspect of the present invention, a method for treating a target surface or air as defined in claim 7 is provided.

In another aspect of the present invention, a surface treated by a method of the present invention is provided.

In still another aspect of the present invention, an article of manufacture as defined in claim 11 is provided.

In yet still another aspect of the present invention, an ionic liquid mixture comprising three or more different ionic liquid components is provided.

### DETAILED DESCRIPTION OF THE INVENVTION

### Definitions

"Consumer product" as used herein refers to a material that is used by a user (i.e., a consumer) in, on or around their person, house (such as kitchen surfaces, bathroom surfaces, carpets, floors, windows, mirrors and countertops), car (such as automobile interiors, automobile exteriors, metal surfaces and windshields), and other personal or household articles (such as dishware, fabrics, cookware, utensils, tableware and glassware). "Consumer product composition" may also include the material used by institutional users (such as hotels, restaurants, offices) or by service providers (such as commercial dry cleaners and janitorial services).

"Industrial product" as used herein refers to a material that is used in a commercial process of making an article. Nonlimiting examples include degreasing compositions for degreasing articles, such as metals; and textile treating compositions for processing and/or finishing textiles into fabric articles, such as garments, draperies.

"Treating" as used herein refers to a composition or a process for cleaning, refreshing or maintaining the target surface or air. For example, "refreshing" includes the processes of removing the wrinkled or worn appearance from a fabric article, or imparting a pleasant odor to a fabric article, air, or a hard surface.

### Ionic Liquids

"Ionic liquid" as used herein refers to a salt that is in a liquid form at room temperature, typically about 20-25°C. The ionic liquid has a melting temperature of about 40°C or less. Typical anionic components of these salts include, but are not limited to, methylsulfate, PF₆⁻, BF₄⁻, or halide.

It should be understood that the terms "ionic liquid", "ionic liquids", and "IL" refer to ionic liquids, ionic liquid composites, and mixtures (or cocktails) of ionic liquids.

Some of the properties that ionic liquids possess and make them attractive alternatives to conventional solvents include:
a) ionic liquids have a broad liquid range; some ionic liquids can be in the liquid form down to -96°C, and others can be thermally stable up to 200°C; this permits effective kinetic control in many organic reactions;
b) ionic liquids have no effective vapor pressure, thus, they are easy to handle and they reduce the safety concerns where volatility could be an issue;
c) ionic liquids are effective solvents for a broad range of organic and inorganic materials due to their high polarity;
d) ionic liquids are effective Bronsted/Lewis acids;
e) ionic liquids can be tuned to the specific application/chemistry desired, for example, they can be selectively made to have properties ranging from hydrophilic to hydrophobic.

By virtue of their high polarity and charge density, ionic liquids have unique solvating properties, and are being used in a variety of applications. These applications include in organic synthesis as a green solvent, in electrochemistry (batteries, electroplating), in novel materials science (liquid crystals, gels, rubbers), and as novel membranes in fuel cells and separations.

Examples of ionic liquids suitable for use herein include, but are not limited to, butylmethylimidazolium hexafluorophosphate: 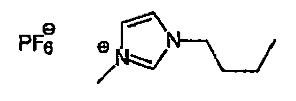 and numerous analogs having varied counterions, alkyl chain lengths, and alternative ring structures such as pyridium. These variables can be adjusted and mixed such that properties of the ionic liquids can be customized for specific applications. These customized ionic liquids have been referred to as "designer solvents".

The ionic liquid has the formula I: 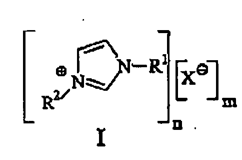 wherein R¹-R² are selected from among the group consisting of linear or branched, substituted or unsubstituted, alkyl, aryl, alkoxyalkyl, alkylenearyl hydroxyalkyl, or haloalkyl; X is an anion; and m and n are chosen to provide electronic neutrality; the resulting salt is a liquid at about 40°C or less. Nonlimiting examples of X include methylsulfate, PF₆⁻ , BF₄⁻, or halide. The R and X moieties may be varied so as to provide the desired solvating properties, viscosity, melting point, and other properties, for the intended application.

### Cocktails of Ionic Liquids

As described above in formula I each ionic liquid may comprise an anionic IL component and a cationic IL component When the ionic liquid is in its liquid form, these components are freely associating with one another (i.e., in a scramble). A "cocktail of ionic liquids", as the term is used herein, comprises at least three different and charged IL components, wherein at least one IL component is cationic and at least one It component is anionic. Thus, the pairing of the cationic and anionic IL, components in a cocktail would result in at least two different ionic liquids.

The cocktails of ionic liquids may be prepared either by mixing individual ionic liquids having different IL components, or by preparing them via combinatorial chemistry.

It is noted that ionic liquids especially lend themselves to preparation via combinatorial chemistry. For example, the following imidazolium based ionic liquid cocktail can be prepared, combinatorially, from three individual IL components (the alkylated imidazole, the alkyl halide, and the anionic charged counterion, such as a halide ion). The following illustrates how the combinatorial chemistry results in a cocktail of ionic liquids.

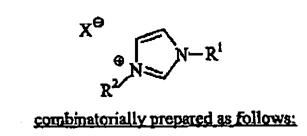

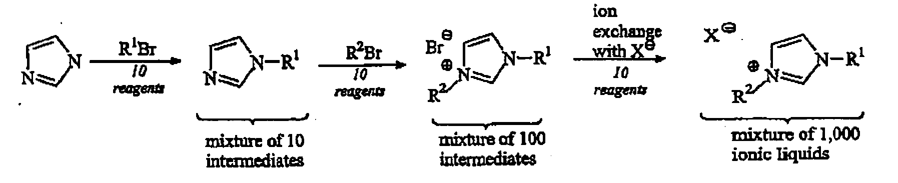

First, the imidazole moieties interact with ten different species of R¹Br to produce a mixture of ten different alkylated imidazole cations and Br⁻ counterions. Then, the alkylated imidazole cations can interact with ten different species of R²Br to produce a mixture of a hundred different alkylated imidazole cations and bromine counterions. This mixture can further interacts with ten different species of X⁻ anions to produce a mixture of 1000 ionic liquids. The R¹, R² and X moieties can be selected from those substituents and anions disclosed in formula I.

Ionic liquid mixtures or cocktails are highly advantageous because the plurality of functional groups and counterions impart varying degrees of hydrophobicity or hydrophilcity, as well as varying degrees of other aspects of solvating power. Such mixture or cocktail would be more effective in its interactions with mixtures of stains/substrates that may be present on a target surface that is being treated with this mixture or cocktail. For example, a burnt-on lasagna residue/stain on a casserole dish may comprise a heterogeneous mixture of protein/starch/lipids, a substantial portion of which may have become polymerized. Accordingly, the plurality of charged IL components in an ionic liquid cocktail is highly efficient in interacting and removing such a stain.

### Compositions Containing Ionic Liquids

The ionic liquids can be present in various compositions suitable for use in applications disclosed above in any desired effective amount, depending on the nature of the intended application. Typically, the ionic liquids are present in an amount ranging from about 0.1% to about 99.9%, preferably from about 1% to about 85 %, and more preferably from about 5% to about 75%, by weight of the composition. In some embodiments, the ionic liquids comprise at least about 50% by weight of the composition.

Many ionic liquids are hygroscopic, thus, may contain appreciable amounts of water (referred to herein as the "innate water") ranging from about 0.01% to about 50% by weight of the ionic liquid. It should be noted that "free water" may be added in making the composition of the present invention. A person of ordinary skill in the art would recognize that once the components (e.g., innate water and free water) are mixed in a composition, the components can no longer be distinguished by their origin and will be reported in totality as percentage of the overall composition. Thus, the compositions of the present invention may comprise water, regardless of its origin, ranging from about 0.01% to about 50%, preferably from about 1% to about 40%, more preferably from about 5% to about 30% by weight of the composition.

The IL-containing compositions may be formulated in the form of liquid, gel, paste, foam, or solid. When the composition is in the solid form, it can be further processed into granules, powders, tablets, or bars.

The ionic liquid compositions may also comprise adjunct ingredients commonly used in air or surface treating compositions. When present, an adjunct ingredient may comprise from about 0.01 to about 10%, preferably from about 0.1 to about 5% by weight of the composition.

Suitable adjunct ingredients may be selected from the group consisting of enzymes, bleaches, surfactants, perfumes, co-solvents, cleaning agents, antibacterial agents, antistatic agents, brighteners, dye fixatives, dye abrasion inhibitors, anti-crocking agents, wrinkle reduction agents, wrinkle resistance agents, soil release polymers, sunscreen agents, anti-fade agents, builders, sudsing agents, composition malodor control agents, dyes, colorants, speckles, pH buffers, waterproofing agents, soil repellency agents, and mixtures thereof.

Examples of suitable adjunct ingredients are disclosed in U.S. 6,488,943, Beerse et al.; U.S. 6,548,470, Buzzaccarini et al.; U.S. 6,482,793, Gordon et al.; U.S. 6,573,234, Sivik et al.; U.S. 6,525,012, Price et al.; U.S. 6,566,323, Littig et al.; U.S. 6,090,767, Jackson et al.; U.S. 6,420, 326, Sherry at al.

Typical examples of enzymes include proteases, amylases, lipases, and mixtures thereof When present, the enzymes may comprise from about 0.01% to about 10%, preferably from about 0.1 % to about 5% by weight of the composition.

Typical examples of co-solvents include linear or branched C1-C10 alcohols, diols, and mixtures thereof. Co-solvents such as ethanol, isopropanol, propylene glycol are used in some of the compositions of the present invention.

### Low-Viscosity Ionic Liquids And Cocktails

Typically, ionic liquids have high viscosities (greater than about 1000 mPa· s) at room temperature. The high viscosities can be problematic in formulating the composition and in applicability. Therefore, the present invention is directed to ionic liquids or cocktails of ionic liquids (undiluted with adjuncts, co-solvents or free water) which have viscosities of less than 750 mPa · s, preferably less than 500 mPa · s, as measured at 20°C. In some embodiments, the viscosity of undiluted ionic liquids are in the range from 0.1 to 400 mPa · s, preferably from 0.5 to 300 mPa· s, and more preferably from 1 to 250 mPa· s.

The viscosities of the ionic fluids and compositions containing them is measured on a Brookfield viscometer model number LVDVII+ at 20°C, with Spindle no. S31 at the appropriate speed to measure materials of different viscosities. The measurement is done at a speed of 12 rpm to measure products of viscosity greater than about 1000 mPa · s; 30 rpm to measure products with viscosities between about 500 mPa · s to about 1000 mPa · s; 60 rpm to measure products with viscosities less than about 500 mPa · s. The undiluted state is prepared by storing the ionic liquids or cocktails in a desiccators containing a desiccant (e.g. calcium chloride) at room temperature for at least about 48 hours prior to the viscosity measurement. This equilibration period unifies the amount of innate water in the undiluted samples.

### New Uses for Ionic Liquids in Compositions for Consumer & Institutional Uses

Applicants have found, surprisingly, that ionic liquids can be added to surface treating compositions to enhance their cleaning and care benefits. Such benefits include but are not limited to soil penetration and removal from treated surfaces, or modification of the aesthetic properties of fabrics and fibers. Surfaces may include hard surfaces found in kitchen, bath, automobile, and the like, and soft surfaces comprising fibers, textiles, fabrics or fabric articles, commonly found in clothing, drapery, linen, carpet, and the like.

Applicants have also found that ionic liquids can also be used advantageously in air treating compositions.

Without wishing to be bound by theory, it is believed that the fundamental chemical and/or physical properties on ionic liquids can be used advantageously in the surface or air treating compositions. In one aspect, ionic liquids have a high solubilizing ability, due to their high polarity and charge density; thus, ionic liquids can be an effective solvent for soils. Therefore, composition containing ionic liquids exhibit enhanced soil removal ability, compared to similar compositions without the ionic liquids. In another aspect, the functional groups and counterions of the ionic liquids can be varied such that the resulting ionic liquids are "tuned" to the characteristics of the target soil or surface. For example, the functional groups can be selected such that the resulting ionic liquids have the desired degree of hydrophilicity or hydrophobicity to interact more strongly or preferentially with the target soil or surface. The mechanisms by which ionic liquids can effectively interact with soil or substrates include, but are not limited to, charge transfer, ion exchange, van der Waals forces, and hydrogen bonding. In yet another aspect, the effective solvating property of the ionic liquids enables them to dissolve certain polymeric materials, which are soluble in few if any solvent media. Examples of such hard-to-dissolve polymers include, but are not limited to, biofilms, baked-on or cooked-on soils, polymerized soils, and the like.

In fabric cleaning and/or treating applications, ionic liquids provide high polarity without the detrimental effects of water. For example, water can causes damages to certain fabrics; the damages include shrinkage, dye loss, shape loss, and wrinkles, etc.

Additionally, the nucleophilic and protic nature of water can lead to undesirable effects when formulating compositions intended for treating fabrics or similar soft surfaces. For example, water's ability to swell and hydrogen bond to cellulose can lead to increased abrasion and shrinkage of fabrics. Ionic Liquids can be tailored or selected to be non-nucleophilic and/or aprotic such that they would not have these adverse effects on cellulosic fibers or fabrics.

In still another aspect, the ionic liquids are non-volatile and nonflammable, and have high thermal stability; as such, they are especially suitable for use in surface or air treating compositions for both safety and aesthetic reasons. It is often undesirable to have chemical vapors or low flash points associated with compositions used in a consumer, industrial or institutional setting. It is also undesirable to have compositions that will leave unsightly streaks on surfaces treated by them. Commonly used organic cleaning solvents tend to have chemical vapors that may be toxic, flammable, or malodorous. Other commonly used compositions may leave unsightly or streaky residue on the treated surfaces, thus, they need to be removed (e.g., by wiping, rinsing, and the like) from the surfaces after application. In contrast, ionic liquids have essentially no vapor pressure (i.e., no detectable vapor pressure at or near room temperature); compositions using ionic liquids as the solvents or the active ingredients would avoid the problems associated with chemical vapors, thus, are highly advantageous. Additionally, such compositions can be used as a leave-on product and produce aesthetically pleasing results on the treated surfaces.

Thus, the unique and customizable physical and chemical properties allow ionic liquids to overcome several problems that persist in prior art compositions for treating soft or hard surfaces or air.

Accordingly, the present invention relates to compositions, consumer products, industrial products, and methods of use the same in following applications: dish/food cleaning, home care (kitchen/bath), biofilm removal, dry-cleaning (home & commercial), laundry (pretreatment, cleaning, and fabric care), textile processing & finishing, car care (interior and exterior), industrial degreasing, and air care.

The ionic liquid may be used in these applications or products as a pure solvent (i.e. as a pure, undiluted ionic liquid or ionic liquid composite); as a co-solvent in conjunction with water or other organic solvents; or as an additive where the continuous phase is water or another solvent (e.g. linear or cyclic siloxanes, halocarbons). Various adjunct ingredients may be incorporated into such compositions.

The ionic liquids or compositions containing them may be delivered to the target surface or air as a liquid or liquid composition via delivery means such as pumps, sprays, and the like. The ionic liquids or compositions containing them may also be delivered via a sheet substrate (such as a wipe made of woven or nonwoven material), a cellular substrate (such as a sponge or a foam), or like substrates. Additionally, the ionic liquids or compositions containing them may be incorporated/deposited into inert porous support materials, which can be made into the form of powders, tablets, and the like.

### Home Care

Certain soils on hard surfaces around the home are extremely difficult to remove and are not adequately treated or removed with via conventional cleaning formulations. These soils can include food soils, outdoor soils, automobile soils, etc. which may be found in the kitchen, bathroom, in and around the toilet, on furniture, and other locations as well.

Ionic liquid compositions may be in the form of a liquid, which can be applied to the target surface as a liquid spray, as an aerosol spray, or as a pour-on liquid, which can be poured onto the target surface directly or indirectly via a substrate such as a fibrous web substrate (made by woven, nonwoven or knitted technologies), a pulp-based substrate (made by air-felt or wet-laid technologies, including paper towels, tissues), a sponge, or a foam substrate. Another mode of use would be to incorporate ionic liquid compositions into or onto these substrates (e.g. impregnated in a wipe or a mitten), which would alleviate residue problems in those applications where complete drydown is needed.

Ionic liquids properties are particular in biofilm removal in home care applications. A biofilm comprises a high cell density community of microbial organisms immobilized on a surface; and typically, the microbes are embedded in a polysaccharide matrix. Biofilms are known to be extremely tenacious and resistant to treatment with conventional antimicrobial agent. Even with extremely aggressive cleaning agents (e.g. chlorine bleach), biofilms are not removed from or cleaned off the target surface. Since the ionic liquids are effective solvents for many organic materials, they exhibit the ability to dissolve polysaccharides. Thus, compositions containing ionic liquids are useful in cleaning and/or removing biofilms, mildew, and other microbe-containing soils, on hard and soft surfaces.

Moreover, the cationic moieties of ionic liquids can be quaternary alkylammonium or alkylphosphonium groups, which are believed to have germicidal properties. Thus, an ionic liquids containing one or more of these cationic moieties would interact with microbial organisms as a biocide to provide sanitizing benefits. Such cleaning and biocidal functions can also be applied to non-domestic settings, such as in institutions where sanitization as well as soil removal are of great importance, e.g., hospitals or restaurants.

### Dish Cleaning And Dishcare

Ionic liquid and compositions may also be used to clean certain stubborn food stains on dishware, tableware and cooking utensils. For example, they may be used to effectively pretreat burnt-on or baked-on soils, which are nearly impossible to remove except with high heat or high mechanical energy (e.g., rigorous scrubbing). Without wishing to be bound by theory, it is believed that the ability of ionic liquids to dissolve polymeric or polar substances would be effective in cleaning and/or removing such stubborn soils. Ionic liquids are particularly effective for removing polymerized grease, which arises from grease or oil that has been baked on or burnt on during cooking; to make matters even worse, the polymerized grease is commonly built up over a long period of time via repeated use and ineffective cleaning in between uses. It is found that ionic liquid compositions are more effective than even the most powerful organic solvents or organic solvent mixtures in removing polymerized grease. For the treatment of the variety of food soils found in most kitchens, compositions comprising ionic liquid mixtures with co-solvents would be desired.

### Laundry

Because of ionic liquids' solvency powers, they have surprising advantages for laundry detergent formulations. In one aspect of the present invention, ionic liquids provide pretreat benefits especially against stains that traditional solvents and surfactants are ineffective. For example, motor oil stains and "heterogeneous" body soil stains which comprise mixtures of particulate, lipids, protein, etc. For this application, the ionic liquid may be a neat liquid or in a composition, and can be applied either as a pretreat product or as an additive to a handwash or machine wash laundry detergent.

In another aspect of the invention, ionic liquid may provide formulation benefits to heavy-duty laundry (HDL) detergents. There are many ingredients that cannot be formulated into laundry detergents due to their chemical or physical instability and/or incompatibility with other ingredients, resulting in phase separation, precipitation, etc. from the laundry detergent. These "difficult to incorporate" ingredients include certain anionic polymers and certain bleach ingredients (for example, polycarboxylates). The solvency, polarity and adjustable hydrophilicity and/or hydrophobicity of ionic liquids makes it easier to formulate with these ingredients.

### Dry-cleaning, Non-aqueous cleaning, and Special Fabric Care

Ionic liquid and compositions containing them containing them are particularly useful in fabric cleaning applications involving dry-clean-only or delicate fabrics. As mentioned before, ionic liquid compositions avoid the damaging effects of water, while still providing the high polarity needed to dissolve polar stains (such as food, beverage, and particulates) that are not very responsive to conventional dry cleaning solvents.

The ionic liquid can be used as a pretreating solvent or as the primary cleaning solvent. Most conventional pretreating or dry cleaning solvents are either water-based or volatile organic compound (VOC)-based. On one hand, ionic liquids do not have the detrimental effects of water on these delicate fabrics; on the other hand, ionic liquids also do not have the health and safety issues relating to volatility of organic solvents.

Moreover, the efficacy of the ionic liquids would be even greater for stains which can have ionic exchange, or which do not dissolve in water or organic solvents. For example, ionic liquids may interact with proteins to render them more soluble in silicones solvents such as decamethylcyclopentasiloxane (D5). Applications would include commercial dry-cleaning, home dry-cleaning appliances, or for "home-dry cleaning" kits (e.g. Dryel^{®}).

In addition, ionic liquids may enable bleaching to take place in the dry-cleaning systems by allowing charge transfer/formation of ionic intermediates to take place. Further, in systems involving solvent recycle, ionic liquids could allow for easier cleanup and recycling of solvent.

### Textile Processing And Home Fabric Care

Ionic liquid and compositions containing them are also useful in fabric treating applications, especially for cellulose-based fabrics, such as cotton.

Without wishing to be bound by theory, it is believed that the ability of ionic liquids to dissolve cellulose may facilitate certain crystal structure changes of cellulose; such changes have been shown to improve the quality of textiles. Cellulose is insoluble in almost everything. Moreover, it is difficult or expensive to induce changes in cellulose without resorting to harsh chemical treatments.

Applications would include consumer fabric care products intended for in-home use, or industrial fabric treatment products intended for the textile processing and finishing industry. For example, ionic liquids may find consumer application in the form of an ironing-aid composition, which the consumer would spray on the fabric surface, then iron it. In the textile processing industry, ionic liquids compositions may be used as a bath or a mist to induce beneficial changes to textiles or fibers. Ionic liquids may also be used as a "primer" that allows other textile actives or processes to be applied to the textiles or fibers. In any case, the fabric, textiles, or fibers treated with ionic liquid compositions exhibit a more functionally or aesthetically pleasing appearance, as well as other benefits, such as durable press benefit, antiwrinkling benefit, antistatic benefit, fiber strengthening benefit, antishrinkage benefit, and like fabric care benefits.

Moreover, since ionic liquids have no vapor pressure, the baths would produce no chemical vapor and raises no VOC safetyor environmental issues for the industrial users.

### Car Care

Certain soils found on car interiors and exteriors are extremely difficult to remove, especially when they have been baked-on due to engine heat and or exposure to the sun. Such soils may include tar, dead insects, grease, soot, bird droppings, food or drink spills. Ionic liquids can be selected to penetrate and assist the removal of such soils. Moreover, unlike organic solvents, the chemical structures of the ionic liquids can be "tuned" so as to not damage the surface being treated, for example, the finish of car exteriors. Applications of the compositions may be in the form of sprays, or wipes impregnated with the IL compositions, or other forms known in the art for delivering liquid compositions. By virtue of their inertness, ionic liquid may also fmd use as automobile antifreeze compositions.

### Air Care

Ionic liquids can also find advantageous uses in air care compositions or devices. In one example of the present invention, ionic liquids may be used as electrostatic precipitators, due to the essentially zero vapor pressure of the ionic liquids. In another example of the present invention, ionic liquids may offer advantages as the solvent in cyclone-based air samplers. Additionally, ionic liquids could help remove charged particulates (soot, etc) from air via charge transfer mechanisms/association, thus, removing or reducing the need for expensive substrate/fiber-based technologies, and increasing the efficiency via increased throughput since no high pressure is needed to pump air through a filter.

### Industrial decreasing

In many industrial applications, grease - especially grease that has become polymerized due to high heat and/or friction present in machinery - is a major problem. Cleaning is typically done with organic solvents or high pressure steam. Ionic liquids or compositions containing them are highly advantageous in such applications because they are effective in removing stubborn grease and are safer to use than the use of volatile chemicals or high-pressure steam.

### Examples

The following are non-limiting examples of consumer product compositions containing ionic liquids.

| | 1 | 2 | 3 | 4 | 5 | 6 | 7 |
|---|---|---|---|---|---|---|---|
| Ionic Liquids | 50 | 50 | 90 | 90 | 95 | 95 | 98 |
| Aesthetic Agents¹ | 1 | 2 | 1 | 1 | 1 | 1 | 1 |
| Enzymes² | 2 | - | - | 1 | - | - | - |
| Adjuncts³ | 10 | 5 | 5 | - | - | - | - |
| Co-solvent⁴ | - | 5 | 2 | - | - | - | - |
| Water | balance | balance | balance | balance | balance | balance | balance |

| | | | | | | | |
|---|---|---|---|---|---|---|---|
| 1. aesthetic agents may be selected from among the group consisting of dyes, colorants, speckles, perfumes and mixtures thereof. 2. enzymes may be selected from among the group consisting of proteases, amylases, lipases, and mixtures thereof. 3. adjuncts may be selected from among the group consisting of surfactants, enzymes, bleaching agents, preservatives and mixtures thereof. 4. co-solvents may be selected from among the group consisting of ethanol, isopropanol, propylene glycol, and mixtures thereof | | | | | | | |

Any of the aforementioned compositions may be impregnated onto one or both sides of an absorbent substrate to afford a "wipe" for use in certain applications. Said absorbent substrate may be formed from any woven or nonwoven fibrous webs, or foam webs. Said absorbent substrate should have sufficient wet strength to hold an effective amount of the ionic liquid containing composition.

## Claims

1. A surface or air treating composition comprising an ionic liquid, wherein the ionic liquid has a viscosity of less than 750 mPa·s as measured at 20°C in its undiluted form, measured on a Brookfield viscometer model number LVDVII+, with spindle 531 at a speed of 12 rpm to measure products of viscosity greater than about 1000 mPa·s; 30 rpm to measure products with viscosities between about 500 mPa·s to about 1000 mPa·s; 60 rpm to measure products with viscosities less than 500 mPa·s. and wherein the ionic liquid is a liquid at 40°C or less and has the general formula: 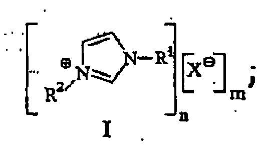 wherein R¹-R² are selected from among the group consisting of linear or branched, substituted or unsubstituted, alkyl, aryl, alkoxyalkyl, alkylenearyl hydroxyalkyl, or haloalkyl; X is an anion; and m and n are chosen to provide electronic neutrality wherein the composition is a laundry detergent, a dish cleaning detergent, a hard surface cleaning composition, a dry cleaning composition, an air care composition, a car care compositions, a textile treating compositions, or an industrial degreasing composition.

2. The composition according to any of the preceding claims wherein the ionic liquid comprises from 0.1% to 99.9 % by weight of the composition.

3. The composition according to any of the preceding claims wherein the composition further comprises an adjunct ingredient selected from the group consisting of cleaning agents, perfumes, enzymes, bleaching agents, surfactants, aesthetic agents, water, co-solvents, and mixtures thereof.

4. The compositions according to any of the preceding claims wherein the composition is in a form selected from the group consisting of solid, liquid, gel, paste, foam, and mixtures thereof.

5. The composition according to any of the preceding claims wherein the composition is in a solid form selected from the group consisting of granules, powders, tablets, bars and mixtures thereof.

6. The composition according to any of the preceding claims wherein the laundry detergent is selected from the group consisting of heavy duty laundry detergents, pretreating compositions, and combinations thereof.

7. A method for treating a target surface or air comprising the step of:
contacting the target surface or air with a composition according to any of the preceding claims.

8. The method according to claim 7 wherein the target surface is selected from the groups consisting of soft surfaces, hard surfaces, and combinations thereof.

9. The method according to any of claims 7 or 8 wherein the soft surfaces are selected from the group consisting of fabric articles, textiles, fibers, and combinations thereof; and the hard surfaces are selected from the group consisting of dishware, cookware, utensils, glassware, countertops, bathroom surfaces, kitchen surfaces, floors, windows, automobile interiors, automobile exteriors, metal and mixtures thereof

10. A surface treated by the method according to any of claims 7 or 9.

11. An article of manufacture comprising a substrate and an ionic liquid according to any of claims 1 to 6.

12. The article according to claim 11 wherein the substrate is selected from the group consisting of a woven fibrous substrate, a non-woven fibrous substrate, a knitted fibrous substrate, a pulp-based air-felt substrate, a pulp-based wet-laid substrate, a foam, a sponge, and combinations thereof.

## Patentansprüche

1. Zusammensetzung zur Behandlung von Oberflächen oder Luft, umfassend eine ionische Flüssigkeit, wobei die ionische Flüssigkeit eine Viskosität von weniger als 750 mPa·s besitzt, gemessen bei 20 °C in ihrer unverdünnten Form, gemessen auf einem Brookfield-Viskosimeter, Modell Nummer LVDVII+, mit Spindel S31 bei einer Geschwindigkeit von 12 U/min zum Messen von Produkten mit einer Viskosität von mehr als etwa 1000 mPa.s; 30 U/min zum Messen von Produkten mit Viskositäten zwischen etwa 500 mPa.s und etwa 1000 mPa.s; 60 U/min zum Messen von Produkten mit Viskositäten von weniger als 500 mPa.s, wobei die ionische Flüssigkeit eine Flüssigkeit bei 40 °C oder weniger ist und die folgende allgemeine Formel besitzt: 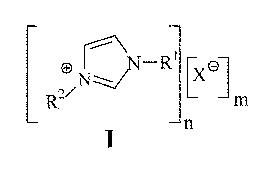 worin R¹-R² ausgewählt sind aus der Gruppe bestehend aus linearem oder verzweigtem, substituiertem oder nichtsubstituiertem Alkyl, Aryl, Alkoxyalkyl, Alkylenarylhydroxyalkyl oder Haloalkyl; X ein Anion ist; und m und n so gewählt sind, dass sie elektronische Neutralität liefern; wobei die Zusammensetzung ein Wäschewaschmittel, ein Geschirrreinigungsmittel, eine Zusammensetzung zum Reinigen harter Oberflächen, eine Zusammensetzung zur chemischen Reinigung, eine Luftpflegezusammensetzung, eine Autopflegezusammensetzung, eine Textilbehandlungszusammensetzung oder eine Zusammensetzung zur industriellen Entfettung ist.

2. Zusammensetzung nach einem der vorstehenden Ansprüche, wobei die ionische Flüssigkeit von 0,1 Gew.-% bis 99,9 Gew.-% der Zusammensetzung umfasst.

3. Zusammensetzung nach einem der vorstehenden Ansprüche, wobei die Zusammensetzung ferner einen Zusatzbestandteil umfasst, ausgewählt aus der Gruppe bestehend aus Reinigungsmitteln, Duftstoffen, Enzymen, Bleichmitteln, Tensiden, Ästhetikmitteln, Wasser, Cosolvents und Mischungen davon.

4. Zusammensetzung nach einem der vorstehenden Ansprüche, wobei die Zusammensetzung in einer Form vorliegt, die ausgewählt ist aus der Gruppe bestehend aus Feststoff, Flüssigkeit, Gel, Paste, Schaum und Mischungen davon.

5. Zusammensetzung nach einem der vorstehenden Ansprüche, wobei die Zusammensetzung in einer festen Form vorliegt, ausgewählt aus der Gruppe bestehend aus Granalien, Pulvern, Tabletten, Stückformen und Mischungen davon.

6. Zusammensetzung nach einem der vorstehenden Ansprüche, wobei das Wäschewaschmittel ausgewählt ist aus der Gruppe bestehend aus Vollwaschmitteln, Vorbehandlungszusammensetzungen und Kombinationen davon.

7. Verfahren zum Behandeln einer Zieloberfläche oder von Luft, umfassend den folgenden Schritt:
Inkontaktbringen der Zieloberfläche oder der Luft mit einer Zusammensetzung nach einem der vorstehenden Ansprüche.

8. Verfahren nach Anspruch 7, wobei die Zieloberfläche ausgewählt ist aus der Gruppe bestehend aus weichen Oberflächen, harten Oberflächen und Kombinationen davon.

9. Verfahren nach einem der Ansprüche 7 oder 8, wobei die weichen Oberflächen ausgewählt sind aus der Gruppe bestehend aus Stoffartikeln, Textilien, Fasern und Kombinationen davon und die harten Oberflächen ausgewählt sind aus der Gruppe bestehend aus Geschirr, Kochgeschirr, Geräten, Glaswaren, Arbeitsplatten, Badezimmeroberflächen, Küchenoberflächen, Böden, Fenstern, Automobilinterieurs, Automobilexterieurs, Metall und Mischungen davon.

10. Oberfläche, behandelt nach dem Verfahren nach einem der Ansprüche 7 oder 9.

11. Herstellungsartikel, umfassend ein Substrat und eine ionische Flüssigkeit nach einem der Ansprüche 1 bis 6.

12. Artikel nach Anspruch 11, wobei das Substrat ausgewählt ist aus der Gruppe bestehend aus einem Gewebefasersubstrat, einem Vliesfasersubstrat, einem Strickfasersubstrat, einem zellstoffbasierten Luftfilzsubstrat, einem zellstoffbasierten Wetlaid-Substrat, einem Schaum, einem Schwamm und Kombinationen davon.

## Revendications

1. Composition de traitement de surface ou d'air comprenant un liquide ionique, dans laquelle le liquide ionique a une viscosité de moins de 750 mPa·s telle que mesurée à 20 °C dans sa forme non diluée, mesurée sur un modèle de viscosimètre Brookfield numéro LVDVII+, avec un mobile cylindrique S31 à une vitesse de 12 tr/min pour mesurer des produits de viscosité supérieure à environ 1000 mPa.s ; 30 tr/min pour mesurer des produits avec des viscosités comprises entre environ 500 mPa.s et environ 1000 mPa.s ; 60 tr/min pour mesurer des produits avec des viscosités inférieures à 500 mPa.s et dans laquelle le liquide ionique est un liquide à 40 °C ou moins et est de formule générale : 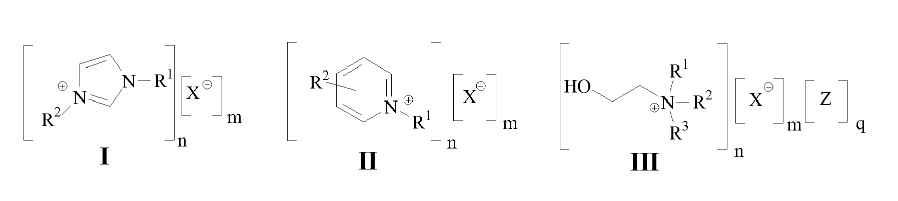 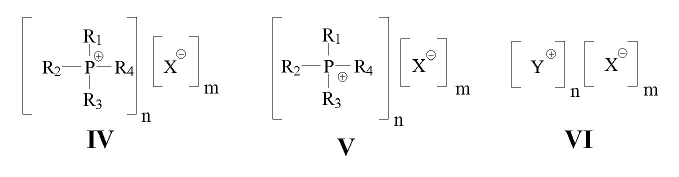 dans laquelle R¹-R² sont choisis parmi le groupe constitué d'alkyle, aryle, alcoxyalkyle, alkylène-aryle hydroxyalkyle, ou haloalkyle linéaire ou ramifié, substitué ou non substitué ; X est un anion ; et m et n sont choisis pour fournir une neutralité électronique où la composition est un détergent pour le lavage du linge, un détergent pour le lavage de la vaisselle, une composition de nettoyage des surfaces dures, une composition de nettoyage à sec, une composition pour le traitement de l'air, une composition pour l'entretien des voitures, une composition de traitement des textiles, ou une composition de dégraissage industriel.

2. Composition selon l'une quelconque des revendications précédentes, dans laquelle le liquide ionique constitue de 0,1 % à 99,9 % en poids de la composition.

3. Composition selon l'une quelconque des revendications précédentes, où la composition comprend en outre un ingrédient additif choisi dans le groupe constitué d'agents de nettoyage, parfums, enzymes, agents de blanchiment, agents tensioactifs, agents esthétiques, eau, co-solvants, et leurs mélanges.

4. Composition selon l'une quelconque des revendications précédentes, où la composition est sous une forme choisie dans le groupe constitué de solide, liquide, gel, pâte, mousse, et leurs mélanges.

5. Composition selon l'une quelconque des revendications précédentes, où la composition est sous une forme solide choisie dans le groupe constitué de granules, poudres, tablettes, pains et leurs mélanges.

6. Composition selon l'une quelconque des revendications précédentes, dans laquelle le détergent pour le lavage du linge est choisi dans le groupe constitué de détergents pour le lavage du linge puissants, compositions de prétraitement, et leurs combinaisons.

7. Procédé pour traiter une surface cible ou de l'air comprenant l'étape consistant à :
mettre en contact la surface cible ou l'air avec une composition selon l'une quelconque des revendications précédentes.

8. Procédé selon la revendication 7, dans lequel la surface cible est choisie dans le groupe constitué de surfaces molles, surfaces dures, et leurs combinaisons.

9. Procédé selon la revendication 7 ou 8, dans lequel les surfaces molles sont choisies dans le groupe constitué d'articles en tissus, textiles, fibres, et leurs combinaisons ; et les surfaces dures sont choisies dans le groupe constitué de plats, batteries de cuisine, ustensiles, verrerie, plans de travail, surfaces de salle de bain, surfaces de cuisine, sols, fenêtres, intérieurs d'automobile, extérieur d'automobile, métal et leurs mélanges.

10. Surface traitée par le procédé selon l'une quelconque des revendications 7 ou 9.

11. Article manufacturé comprenant un substrat et un liquide ionique selon l'une quelconque des revendications 1 à 6.

12. Article selon la revendication 11, dans lequel le substrat est choisi dans le groupe constitué d'un substrat fibreux tissé, un substrat fibreux non tissé, un substrat fibreux tricoté, un substrat de feutre aéré à base de pâte, un substrat par voie humide à base de pâte, une mousse, une éponge, et leurs combinaisons.
